# EUROPEAN PATENT SPECIFICATION

(11) **EP 2 277 993 B1**
(45) Date of publication and mention of the grant of the patent: **02.09.2015**
(21) Application number: 08873385.2
(22) Date of filing: 24.12.2008
(51) Int. Cl.: C12N 5/071

(54) **METHOD FOR PRODUCING ENDOTHELIAL CELLS (VARIANTS)**
VERFAHREN ZUR PRODUKTION VON ENDOTHELZELLEN (VARIANTEN)
PROCÉDÉ DE FABRICATION DE CELLULES ENDOTHÉLIALES (ET VARIANTES)

(30) Priority: 19.03.2008 RU 2008110210
(43) Date of publication of application: 26.01.2011
(73) Proprietor: Obshhestvo S Ogranichennojj Otvetstvennost' Ju "Laboratorija Kletochnykh Tekhnologijj", Moscow 127051 (RU)
(72) Inventor: KISELJOV, Sergejj L'vovich, Moscow 123458 (RU); LAGAR'KOVA, Marija Andreevna, Moscow 119607 (RU)
(74) Representative: Glawe, Delfs, Moll
(86) International application number: PCT/RU2008/000795
(87) International publication number: WO 2009/116893

(56) References cited:
- WO-A1-2007/037682
- WO-A2-03/040319
- US-A1- 2005 191 744
- US-A1- 2005 272 152
- XU MEIFENG ET AL: "In vitro and in vivo effects of bone marrow stem cells on cardiac structure and function.", JOURNAL OF MOLECULAR AND CELLULAR CARDIOLOGY FEB 2007 LNKD- PUBMED:17187821, vol. 42, no. 2, February 2007 (2007-02), pages 441-448, XP5853942, ISSN: 0022-2828
- ZHOU XIAO-YING ET AL: "[Promoting effects of serum-free murine bone marrow endothelial cell conditioned medium on the growth of bone marrow endothelial cells].", SHENG LI XUE BAO : [ACTA PHYSIOLOGICA SINICA] 25 APR 2005 LNKD- PUBMED:15830105, vol. 57, no. 2, 25 April 2005 (2005-04-25) , pages 199-204, XP9152229, ISSN: 0371-0874
- CHEN H-F ET AL: "Derivation, characterization and differentiation of human embryonic stem cells: comparing serum-containing versus serum-free media and evidence of germ cell differentiation.", HUMAN REPRODUCTION (OXFORD, ENGLAND) FEB 2007 LNKD- PUBMED:17071820, vol. 22, no. 2, February 2007 (2007-02), pages 567-577, XP2472443, ISSN: 0268-1161
- HAY DAVID C ET AL: "Direct differentiation of human embryonic stem cells to hepatocyte-like cells exhibiting functional activities", CLONING AND STEM CELLS, vol. 9, no. 1, April 2007 (2007-04), pages 51-62, XP2454568, ISSN: 1536-2302
- GERECHT-NIR S ET AL: "HUMAN EMBRYONIC STEM CELLS AS AN IN VITRO MODEL FOR HUMAN VASCULAR DEVELOPMENT AND THE INDUCTION OF VASCULAR DIFFERENTIATION", LABORATORY INVESTIGATION, NATURE PUBLISHING GROUP, US, vol. 83, no. 12, 1 December 2003 (2003-12-01), pages 1811-1820, XP001203785, ISSN: 0023-6837, DOI: 10.1097/01.LAB.0000106502.41391.F0
- PICK MARJORIE ET AL: "Differentiation of human embryonic stem cells in serum-free medium reveals distinct roles for bone morphogenetic protein 4, vascular endothelial growth factor, stem cell factor, and fibroblast growth factor 2 in hematopoiesis", STEM CELLS, ALPHAMED PRESS, vol. 25, no. 9, 1 September 2007 (2007-09-01), pages 2206-2214, XP008092704, ISSN: 1549-4918, DOI: 10.1634/STEMCELLS.2006-0713 [retrieved on 2007-06-07]
- KIM S ET AL: "Endothelial stem cells and precursors for tissue engineering: Cell source, differentiation, selection, and application", TISSUE ENGINEERING PART B-REVIEWS,, vol. 14, no. 1, 1 March 2008 (2008-03-01), pages 133-147, XP002592955, ISSN: 1937-3368, DOI: 10.1089/TEB.2007.0304
- DATABASE PUBMED MEDLINE [Online] GERECHT-NIR S. ET AL.: 'Human embryonic stem cells as an in vitro model for human vascular development and the induction of vascular differentiation.', XP001203785 Database accession no. 14691299 & LAB INVEST vol. 83, no. 12, December 2003, pages 1811 - 20

## Description

### Field of the Invention

The invention relates to biotechnology in particular to producing endothelial cells from human embryonic stem cells (ESCs) and to the use thereof. Human embryonic stem cells (hESCs) must be considered as a valuable source for regenerative medicine due to their capability to differentiate into any cell type. The endothelial cells produced as a result of differentiation might be widely used in clinical practice and in investigation of differentiation mechanisms. Endothelial cells form not only blood vessel walls but also are able to migrate to different sites within the blood flow. Therefore, they might be used not only for blood vessel regeneration by means of their integration or secretion of growth factors, but serve as carrier for a therapeutic gene or a product delivery. However, for potential clinical usage of differentiated in vitro cells it is necessary to confirm that a desired cellular phenotype would be maintained without returning to the pluripotency.

### Background of the Invention

Embryonic stem cells are produced from a blastocyst inner cell mass that is formed at the stage of a blastocyst growth. The ability of hESCs to differentiate into some specific cell types is under intensive investigation at present. The general aim of such studies is to produce differentiated functional cells. However, these cells can be used for treating man only in case of providing stable cell specialization. Therefore, not only morphological, immunologic and functional properties of a cell as well as genetic and epigenetic ones must be taken into consideration.

Differentiation of ESCs in a certain way is followed by an alteration of the gene expression pattern from genes specific for pluripotency to tissue-specific genes. The expression pattern alteration occurs due to a decay of an expression of key transcription factors participating in maintenance of pluripotency and by the expression of tissue-specific transcription factors. Epigenetic mechanisms regulating gene expression work parallel repressing genes required for pluripotency and activating tissue-specific ones. ESCs are *in vitro* analogues of epiblast cells artificially maintained in a non-differentiated state, they spontaneously differentiate into various cell types when culturing conditions are changed. In vitro spontaneous differentiation of human ESCs can be seen in both long-termed culturing of attached colonies and growing embryoid bodies (EB) in a suspension. Apart from mouse ESCs, not every human ESC line produces embryiod bodies easily, neither endothelial stem cell line produces them from a single cell.

The capability of human ESCs to differentiate into many cell types such as neurons and glia, endothelium, keratinocytes, trophoblasts, cardiomyocytes, osteoblasts, blood cells, hepatocytes, insulin-producing cells and some others has been demonstrated.

It was most difficult to produce entoderm derivatives. In recently published reports on human ESC differentiation into insulin-producing cells (1) and hepatocytes (2), it was shown that in their protein and genetic markers they were identical to mature insulin-secreting cells and hepatocytes. However, the factors determining differentiation into the entoderm are poorly characterized, the markers of an early entodermal differentiation are almost lacking.

Apart from mouse ESCs which are easily differentiated into cardiomyocytes in the absence of a leukemia inhibitory factor (LIF), spontaneous differentiation of human ESCs occurs in different ESC lines in various rates - from spontaneously contracting regions in 70% EBs to complete absence of spontaneous differentiation (3). Cardiomyocytes differentiated from human ESCs have been shown to express transcription factors Nkx 2.5, GATA4 as well as cardiomyocyte specific markers - troponin 1 and an alpha-myosin heavy chain. The cardiomyocyte differentiation in a serum-free medium bypassing the stage of embryoid bodies has been published recently (4). When cultured for a long time the ESCs can undergo an artificial accidental selection. Moreover, it turned out, endothelial stem cells identical in main morphologic characteristics and main genes expression can differ in an epigenetic state in regulatory zones of several genes. This might lead to that cell lines of ESCs would have the predominant ways of differentiation. That is, for example, ESCs would differentiate readily into neurons and poorly into cardiomyocytes. Perhaps, it is the difference in the epigenetic status of human and mouse ESCs that can explain such a different potential for differentiation.

In a normal embryo development a vascular tissue is formed from the mesoderm. Shortly after gastrulation the mesoderm forms as a separate layer between ecto- and endoderm. Endothelial and hematopoietic cells are supposed to have originated from the same mesodermal progenitor - a hemangioblast (5). The progenitor cells potentially capable to produce hematopoietic and endothelial cells were identified in a mouse ESC model (6,7,8) and thereafter in vivo when studying gastrulation of a mouse embryo (9). There are three main technological approaches to study differentiation of ESCs into endothelial and hematopoietic cells. The first one is ESC differentiation into endothelial and hematopoietic cells via the stage of embryoid bodies (10-15). The second one implies a combined culturing of ESCs and stromal cells (16, 17). The third approach involves differentiation of ESCs under two-dimensional conditions (18, 8).

The differentiation of endothelial stem cells via the stage of an embryoid bodies formation is always spontaneous, uncontrolled differentiation. As it has already been mentioned an embryoid body contains cells originating from different germ layers, that is why the number of cells of the required type is low. The number of endothelial cells in embryoid bodies is about 2%. Besides, this approach has some more limitations. For example, it is difficult to observe the cells under a microscope, to do a differentiation analysis of single cells, to isolate cells from cell aggregates in EBs. However, to date it is this approach that has been used and described in many publications. This is due to, in particular, that targeted differentiation of cells requires a special selection of conditions, EBs being a crude simplified model analogue of a developing embryo where there are various cells including those that are required for a research.

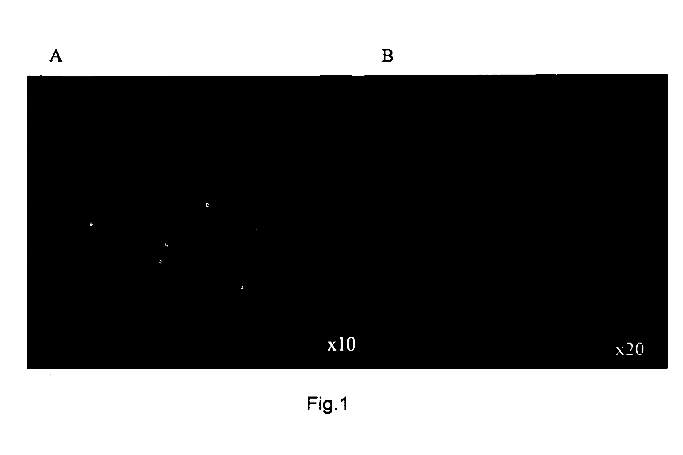

In a reference example it was tested if there are endothelial cells in embryoid bodies produced from human ESC lines available hESM01 and hESM03 lines were analyzed. These lines having been produced in the laboratory before had the structure characteristic for the given cell type, expressed corresponding markers and maintained a normal karyotype (19). When introduced into a suspension culture ESCs formed embryoid bodies with the characteristic structure for the given type structures. An immune histochemical assay of embryoid bodies in 10-12 days of culturing revealed a small amount (not more than 1-5%) of endothelial cells expressing CD31 (Fig.1).

The differentiation of human ESCs into endothelial cells under two-dimensional conditions (bypassing the EB formation stage) was used by the only research group (20). We chose the known technique as a nearest analogue. The technique involves differentiation of human ESCs in the presence of a human Vascular endothelial growth factor (VEGF) on a collagen IV feeder. A selection of endothelial cells was carried out according to the cell size by an infiltration with a pore diameter filter of 40 µm. It is not clear from the article what post-selection portion the endothelial cells accounted for. However, suppose that endothelial cells are related to hematopoietic ones and originate from the same hemangioblast progenitor, a mechanical separation could not ensure a desired cell purity. This method is not likely to be worth using, if there is a necessity to produce a pure population of endothelial cells.

### The Brief Description of the Figures

**Fig.1** Immune histochemical assay of a 10 µm EB cryosection of 12 days culturing.
   A - EBs of an ESM01 line, stained with CD31 antibodies (red); B - EBs of an ESM03 line, stained with CD31 antibodies (red); the nuclei are stained with DAPI (blue). Secondary goat anti-mouse Alexa Fluor 546 antibodies.
**Fig.2** Formation of vascular-like structures and a monolayer in differentiation of human ESCs (8 days). A - a light field. B - immune histochemical staining with CD31 antibodies.
**Fig.3** Viability evaluation of CD31 positive cells after immunomagnetic separation. A - the 5^{th} differentiation day; B - the 7^{th} differentiation day. Living cells (green), dead cells (orange). The picture was taken under a fluorescent microscope.
**Fig.4** Human ESC differentiation into endothelial cells in a two-dimensional system: A - non-differentiated colonies of ESCs on a MEF feeder; B - differentiated derivatives, 3^{rd} culturing day; C - differentiated derivatives, 5^{th} culturing day; D - vascular-like structures, 7^{th} culturing day.
**Fig.5** A - CD31 endothelial cells derived from ESCs, 24 hours after selection (the picture in a light field). B - immune histochemical assay of ESCs after immunomagnetic separation. CD31 expression (red); nuclei are stained with DAPI (blue). Secondary Alexa Fluor 546 antibodies. B - Matrigel Assay demonstrating an endothelial nature of cells selected with immunomagnetic separation.
**Fig.6** RT-PCR assay of VE-cadherin, GATA-2, Flk1, GAPDH, GATA-3, eNOS, CD31 genes in non-differentiated ESCs of the ESM03 line (U), endothelial cells from ESC ESM03 line obtained with immunomagnetic separation (E), and in human umbilical vein endothelial cells (H).
**Fig.7** Results of bisulfite sequencing of the promoter region of the GATA-2 gene from 43 to -187 nucleotides in relation to a transcription start. (A) - human ESCs, (B) - endothelium derived from human ESCs, (C) - HUVECs.
**Fig.8** Results of bisulfite sequencing of the promoter region of the GATA-3 gene from 228 to -115 nucleotides in relation to a transcription start. (A) - from human ESCs, (B) - endothelium derived from human ESCs, (C) - HUVECs.
**Fig.9** Results of bisulfite sequencing of the promoter region of the eNOS gene from 13 to -297 nucleotides in relation to a transcription start. (A) -from human ESCs, (B) - endothelium derived from human ESCs, (C) - HUVECs.
**Fig.10** Alteration of the blood vessel density 30 days after an introduction of endothelial cells: 10a - before introduction, 10b - 30 days after introduction.
**Fig.11** (a) - endothelial cells-free matrigel removed from experimental animals in 8 days, (b) - endothelial cells containing matrigel removed from experimental animals in 8 days.

### Detailed Description of the invention

We set up a task to develop an effective and reproducible method of hESC differentiation into a pure population of functional endothelial cells where alterations of a genetic regulation pattern correspond with mature endothelial cells. Unlike differentiation via the EB stage, a direct differentiation of ESCs is easy to observe and manipulate cells. However, it requires preliminary investigations to determine the differentiation conditions. Moreover, firstly, controlled differentiation must result in preferably desired cell phenotype. Secondly, there must be a possibility to select eligible cell populations, where the process of selection itself does not have to affect cells viability and their differentiation significantly. Another imposed task was to develop a method for separating endothelial progenitors and endothelial cells from differentiated human ESCs.

The lack in literature of the information on human ESC differentiation in a two-dimensional system without EB formation into endothelial cells required differentiation conditions and media to be determined. More than 15 culturing techniques with various combinations of media, sera and growth factors have been tested. For this purpose ESC colonies were placed into Petri plates with growth media of a various composition (Table 1).

**Table 1. The comparison of various media for human ESC differentiation into the endothelium. The comparison was carried out in 8 days of culturing based on an immune histochemical assay.**

| Medium | Alpha-MEM | Alpha-MEM | EGM | EGM | DMEM/F12 | DMEM/F12 | DMEM/ F12 |
|---|---|---|---|---|---|---|---|
| Serum | Hyclone defined | Serum replacement | - | - | Hyclone characterized | Hyclone characterized | KO medium substitute |
| Growth Factors | Activin, VEGF | VEGF, FGF, SCF, EGF | FGF | VEGF FGF | VEGF, FGF, SCF, EGF | VEGF, SCF, bFGF | VEGF, SCF, BMP4, TGFbeta 1, bFGF |
| Feeder | Collagen I | Collagen IV | Collagen IV + Collagen I | Collag en IV | Collagen IV | Collagen IV | Collagen IV |
| Endothelial cells, % | 0-2 | 5-8 | 5-8 | 10-15 | 25-40 | | ≥50 |

During a differentiation process individual cells expressing an early endothelium CD31 marker and a late differentiated endothelium marker (von Willebrand factor (vWF)) were revealed.

Having used various compositions of media in different combinations with growth factors the growth factors providing effective differentiation of human ESCs into endothelial cells have been revealed. Such factors of a protein nature include the stem cell factor (SCF) (manufactured by *Chemicon*)*,* the human vascular endothelium growth factor (VEGF) (manufactured by *Chemicon),* the bone morphogenic protein BMP4, the basic fibroblast growth factor bFGF (manufactured by *Chemicon*)*,* the tumor growth factor TGFbeta.

The proportions of these growth factors depend on a medium composition, the presence or absence of components of uncertain origin in the medium, also they may vary depending on the ESC lines used. The amount of growth factors can be determined by an experimental way.

During the differentiation in a medium containing necessary components for directed differentiation of human ESCs into endothelial cells, we were able to observe alterations both in the cell structure and in the specific expression of endothelial markers. At first, on the 4^{th} day after the induction of the differentiation the CD31 endothelial markers were revealed. Emerging of vascular-like structures coincided with the vWF expression. On the 6-7^{th} days almost 60% of the cells were expressing a CD31 molecule, by the end of the differentiation procedure (12^{th} day) approximately 50% of cells were expressing a CD105 antigen (Table 2). At the same time it must be stated, that the CD133 molecule expression was rather high in non-differentiated hESCs and was reducing in endothelial progenitors during differentiation. A rather high percentage of differentiation into endothelial cells allowed us to carry out a test of a vessel formation without selecting cells.

**Table 2**

| Marker | Differentiation Days | | | | | | |
|---|---|---|---|---|---|---|---|
| | 0 | 2 | 4 | 6 | 8 | 10 | 12 |
| CD31 | - | - | + | + | + | + | + |
| VE-cadherin | - | - | + | + | + | + | + |
| CD133 | + | + | +/- | +/- | +/- | - | - |
| vWF | - | - | - | - | + | + | + |
| CD-105 | - | - | - | - | - | + | + |
| CD-34 | - | - | + | + | + | + | + |

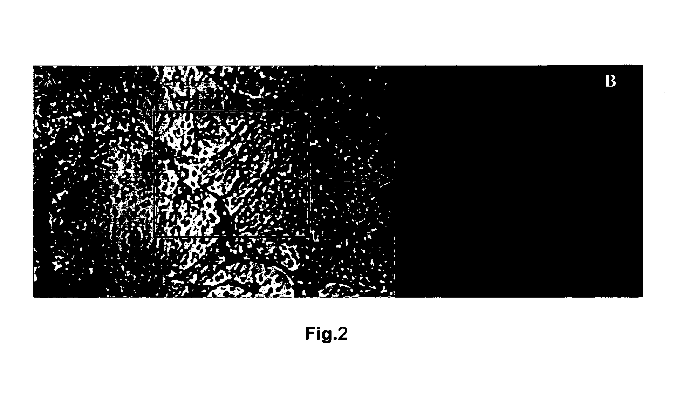

CD31 positive cells appear not only in vascular-like structures but also as a monolayer (Fig.2). According to the investigation findings the balance between vascular-like cells and the monolayer depends on a VEGF concentration.

To separate endothelial cells differentiated from human ESCs, we employed an immunologic selection method using surface or internal markers specific for endothelial cells. For example, in the given case a method of magnetic activated cell sorting (MACS) was used that is an effective technique of magnetic sorting based on the separation of cells on surface antigens (www.miltenyibiotec.com). Such a method enables a fast and effective selection (with the purity of 98% and more) of a needed cell population on surface markers. However, another method can also be used, for instance, fluorescent activated cell sorting (FACS), and any other ones providing cell sorting in dependence on surface or internal markers.

To select an endothelium produced by ESCs in a two-dimensional system we used a CD31 marker. Other endothelial markers such as CD34, vWF or so on can also be employed.

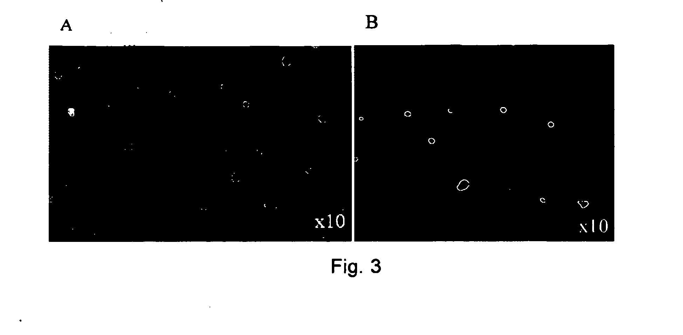

Selection on the presence of CD31 cells was carried out on the 5^{th}-7^{th} differentiation day. The viability of cells selected on the 5^{th} day was noticed to be higher than that of selected on the 7^{th} day of differentiation (Fig.3). It can be explained that on the 7^{th} differentiation day the cells of endothelium have already been forming structures that are rather difficult to be destroyed by a fermentative treatment, with the treatment prolongation and/or destruction of formed intercellular contacts leading to reduction of the viability. Therefore, a selection on the 5^{th} differentiation day under conditions we used can be considered optimal. Using other markers a selection can be performed on the 3^{rd} - 9^{th} days of differentiation.

The cells selected were placed in collagen-covered plates into a medium for endothelial differentiation. They were plated in a high density (not less than 50,000 cells per 1 cm² and more), since the plating in a lower density affected the cell viability.

We suggested two variants of the method to produce endothelial cells from human ESCs.

The first variant of the method for producing endothelial cells from human ESCs involves the following:
1. Differentiation of human ESCs on a substrate feeder and in an artificial medium that provide targeted direct differentiation of human ESCs into endothelial cells with VEGF, SCF, BMP4, bFGF, TGFbeta. As the artificial medium base a mixture of liquid DMEM/F12 with a liquid KO serum substitute is used in the following ratio (V%): the liquid DMEM/F12 - 75-95%, the liquid KO serum substitute - 5-25%, where TGFbeta is introduced into the artificial medium on the 3^{rd}-6^{th} differentiation day.
2. Immunologic selection based on the use of markers specific for the endothelium of the 3^{rd} - 9^{th} days of differentiation.
3. Cultivation of endothelial cells with the plating density not less than 50,000 cells per 1 cm².

The second variant of the method for producing endothelial cells from human ESCs involves the following:
1. Differentiation of human ESCs on a substrate feeder and in a medium that provide targeted direct differentiation of human ESCs into endothelial cells with VEGF, SCF, bFGF. A medium is based on the combination of a liquid DMEM/F12 and a fetal bovine serum in the ratio (V%): the liquid DMEM/F12 - 75-95%, the fetal bovine serum - 5-25%.
2. Immunologic selection based on the use of markers specific for the endothelium of the 3^{rd} - 9^{th} differentiation days.
3. Cultivation of endothelial cells with the plating density not less than 50,000 cells per 1 cm².

In a reference example cells of the hESM01, hESM03, HESM02, 04, H1, HUES7, 8, 9 lines were tested, with the results obtained being alike, that confirms the method's potential for differentiation of any human ESCs.

The ratio of medium components may be optimized for an individual cell line to achieve the maximum efficacy. As a culturing medium other artificial media of known and characterized compositions can be used.

The claimed method in both variants allows to obtain a high yield of cells in the end of a differentiation procedure which have immunologic endothelium markers, most of them are able to participate in formation of vascular-like structures. Moreover, the differentiation was reproduced when using a serum substitute instead of a standard fetal bovine serum (FBS) that is significantly better of the existing differentiation methods. Using an artificial medium free of components of an animal origin makes this method reproducible and independent on a serum composition. However, even in this case approximately a half of cells was of an unknown cell type or an insignificant population of non-differentiated cells. It is important to note that even a small proportion of non-differentiated cells or cells differentiated into various types can contaminate a cell culture. These side populations can have a great impact on a safe and functional efficacy of the culture for therapeutic use or further investigation. Thus, the selection of a specific cell population is of importance.

CD31 has been considered as the best immune marker of endothelial cells, at the same time its expression was noticed very early on the 6^{th} day of differentiation of hESCs, that allowed to use it in magnetic activated cell sorting. However, the usage of other markers (e.g. CD34, vWF) and other selection methods (e.g. FACS) also gave good results.

### Ways of the Invention Implementation

The potential of the method is confirmed by the following examples that demonstrate only individual cases of its implementation.

### Reference Example 1. Culturing Human Embryonic Stem Cells.

Lines of human ESCs were cultured in a medium containing 80% of KnockOut DMEM, 20% of a KO serum substitute, 1 mM of glutamine, 1% non-essential amino acids, 50 U/ml penicillin, 50 µg/ml streptomycin (manufactured by *Invitrogen*), 0.1 mM β- mercaptoethanol (*Sigma*), and 4 µg/ml bFGF (*Chemicon*) on mitomycine C-inhibited (10 µg/ml, *Sigma*) mouse embryonic fibroblasts with the density of 10⁴ cells/cm² in culture plates covered with 0.1 % gelatin (*Merck*)*.* The fibroblasts were prepared from 12 day mouse embryos of the F1 (C57BL/6JxCBA/Ca). hESCs colonies were sub-cultured every 5-6 days of culturing by treating with collagenase IV (200 U/ml, *Invitrogen*) and a mechanic dissection. The plates with non-differentiated colonies of hESCs cultured for 8 days were treated with collagenase IV for 10 minutes, washed out twice with a growth medium and the colonies were collected from a feeder with a micropipette tip by-hand. Pluripotent cell lines, isolated and cultured on a feeder, demonstrated the cell structure typical for ESCs, expressed immune markers characteristic for non-differentiated cells and maintained a normal karyotype. We did not observe even a small portion of CD31-positive cells in non-differentiated colonies.

### Reference Example 2. Differentiation of Human Embryonic Stem Cells into Endothelial Cells.

### A medium for differentiation.

To differentiate cells an artificial medium of the following composition was used: the base - 85% of a liquid DMEM/F12 *(Hyclone* or any equivalent analogue), 15% of a liquid KO serum substitute (*Invitrogen* or any equivalent analogue) with addition of 1% non-essential amino acids, 2 mM L-glutamine, 50 U/ml penicillin, 50 µg/ml streptomycin, 50 ng/ml SCF, 10 ng/ml BMP4, 50 ng/ml VEGF, 10ng/ml activine, 20ng/ml bFGF, 2 ng/ml TGFbeta. The TGFbeta is introduced into the medium on the 4^{th} day of differentiation. The concentrations and combinations of growth factors may be 50-100% different from the outlined ones in dependence on a cell line and individual culturing conditions.

### A procedure of a medium preparation

The liquid DMEM/F12 medium was mixed with the liquid serum substitute, antibiotics and glutamine and stored at +4° C to use thereof. All the other components (growth factors and so on) were added to the medium immediately before adding them to cells. The complete medium was not stored.

### A technique of differentiation

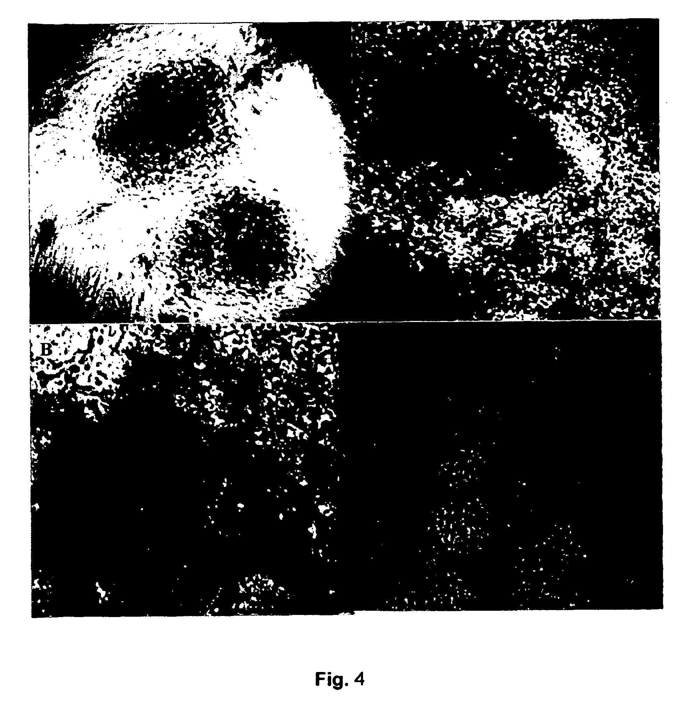

The plates containing non-differentiated hESC colonies that had been cultured for 8 days (Fig. 4A) were treated with collagenase IV for 10 min, washed out with a growth medium twice, then the colonies were collected by-hand with a micropipette tip, cutting them into fragments with the size of 300-500 cells. To carry out the differentiation the ESC colonies were transferred into 35mm Petri plates covered with collagen IV (70 ug/ml) in the medium of the above composition (Fig.4B). On the 4^{th} differentiation day 2 ng/ml of TGFbeta 1 were added to the medium. The day following the introduction of TGFbeta 1 the cells got the structure of differentiated derivatives (Fig. 4C). On the 6^{th} -10^{th} culturing days the colonies contained clusters of vascular-like structures (Fig. 4D).

### Reference Example 3. Differentiation of Human Embryonic Stem Cells into Endothelial Cells.

This example differs from the 2^{nd} one in using a medium based on a mixture of the liquid DMEM/F12 (*Hyclone*) and the fetal bovine serum (*Hyclone,* characterized).

### A medium for differentiation.

To differentiate cells a medium of the following composition was used: the base - 85% of liquid DMEM/F12 *(Hyclone* or any equivalent analogue), and 15% of a fetal bovine serum *(Hyclone* or any equivalent analogue), 2 mM of L-glutamine, 1% non-essential amino acids, 50 U/ml penicillin, 50 µg/ml streptomycin (all of *Invitrogen* or any equivalent analogue), 20 g/ml SCF (*Chemicon* or any equivalent analogue), 10-50 ng/ml VEGF (*Chemicon* or any analogue), 5 ng/ml bFGF (*Chemicon* or any equivalent analogue). Growth factors may vary as mentioned in the example 2. The medium is prepared as described in Example 2.

On the 3^{rd} -6^{th} day the cells got the structure of differentiated derivatives. On the 6^{th}-10^{th} culturing days the colonies contained clusters of vascular-like structures.

### Reference Example 4. Separation of Endothelial Cells.

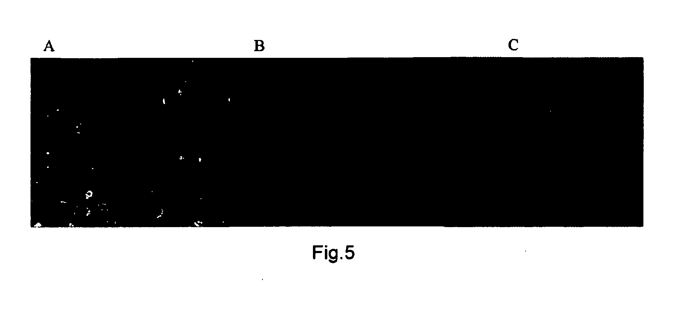

For CD31⁺ cellular selection the cells were separated with treatment of collagenase IV (*Invitrogen* or any equivalent analogue) at the temperature +37° C for 10 min and washed out twice with a phosphate buffered saline solution (PBS) containing 0.5% of a bovine serum albumin (BSA) on the 5^{th}-7^{th} differentiation day. The cells produced were incubated for 30 min together with mouse anti-human CD31 antibodies in the ratio of 10 µg x 10⁶ cells and then with goat anti-mouse IgG conjugated with magnetic beads (*Miltenyi Biotec* or any equivalent analogue) for 15 min at the temperature +8° C. CD31⁺ cells were isolated passing through a filter and a MS column attached to the magnetic block of a Mini-MACS separation unit (*Miltenyi Biotec* or equivalent analogue) to obtain a magnetically retained a fraction of purified CD31⁺ cells. After elution CD31⁺ cells were placed on collagen IV-covered plates with the plating density of 50,000 cells per 1 cm², the cells were grown in an endothelial maintenance medium (Fig. 5A).

### The Industrial Applicability

The purity of a selected population was evaluated by an immunocytochemistry assay. To determine whether CD31⁺ cells eluted from the column are endothelial cells or their progenitors, a part of the cells was subjected to an endothelial function testing - a Matrigel assay. To assess the expression of genes characteristic for the endothelium a semi-quantitative RT-PCR method was applied.

### Immunocytochemistry Assay

The cells were fixed with 100% ice methanol for 5 min or with 4% paraformaldehyde for 10 min at room temperature. The slides were washed in three PBS solutions, 0.1 % Tween 20 and incubated with the following secondary antibodies: Alexa Fluor 546-conjugated goat anti-mouse IgG, Alexa Fluor 488-conjugated goat anti-mouse IgG in the dilution of 1:800. Primary antibodies used were anti-human CD31, anti-human vWF, anti-human CD105, anti-human CD34, anti-human CD133, anti-human Flk-1, anti-human CD14, anti-human CD45 ones. The cells were also stained with DAPI (4',6-diamidino-2-phenylindole). Thereafter the immune-labeled cells or fragments were studied under a fluorescent microscope. To avoid detecting artifacts, we used antibodies of a different specificity from those used to select cells. The cell population purity was more than 90% (Fig. 5B).

### Testing an ability to form vascular-like structures

Testing was carried out as described in (21). Matrigel was added into 24 wells of the plate. 0.5-2x10⁴ cells were put into the wells in 0.5 ml of a growth medium and incubated in 5% CO2 at the temperature +37° C. The cell structure was studied under an inverted light phase-contrast microscope. As it can be seen in Figure 5C, the cells form a vascular-like mesh network characteristic for the endothelium.

### Reverse Transcription PCR (RT-PCR)

A total RNA was isolated from non-differentiated hESCs, CD31 selected endothelial cells and HUVECs (human umbilical vein endothelial cells) using an RNeasy Mini Kit (*Qiagen*). The RT-PCR was carried out with the RNA (1-2 µg), random 6-nucleotide primers (*Promega*), a RevertAid M-MuLV reverse transcriptase and reagents (*Promega*) in concordance with the manufacturer's recommendations. A coding DNA was dissolved in the final volume of 25 ml. The certain volume of the cDNA (1-5 ml) was subjected to PCR amplification using a Taq DNA polymerase and reagents (*Fermentas*)*.* All the RNA samples were able to result in an equal amplification of the GAPDH (glyceraldehyde 3-phosphate dehydrogenase) as an internal control. The sequences of primers for the RT-PCR are given in Table 3. The amplification products were separated in a 1.5% agarose gel.

**Table 3**

| Gene | Sequences of primers |
|---|---|
| VE-cadherin | 5'CCGGCGCCAAAAGAGAGA3' |
| | 5'CACGGACGCATTGAACAAC3' |
| GATA-2 | 5'CCCTAAGCAGCGCAGCAAGAC3' |
| | 5'TGACTTCTCCTGCATGCACT3' |
| GATA-3 | 5'TCACAAAATGAACGGACAGAAC3' |
| | 5'GGGTTAAACGAGCTGTTCTTG3' |
| eNOS | 5'GTGATGGCGAAGCGAGTGAAG3' |
| | 5'CCGAGCCCGAACACACAGAAC3' |
| CD31 | 5'GCTGTTGGTGGAAGGAGTGC3' |
| | 5'GAAGTTGGCTGGAGGTGCTC3' |
| Flk 1 | 5'TGGAGGTGACTGAGTGCAG3' |
| | 5'GACCATGCCAGCATAGCTG3' |
| DPPA3 | 5'TACTCCGTCGAGAGTCTGTAG3' |
| | 5'TTGGGCTTCTAAGACACATGG3' |
| DPPA5 | 5'ACCTGAAAGATCCAGAGGTGT3' |
| | 5'CCGGCTTCATTGCATTGGCT3' |
| OCT4 | 5'CGACCATCTGCCGCTTTGAG3' |
| | 5'CCCCCTGTCCCCCATTCCTA3' |
| NANOG | 5' AGCATCCGACTGTAAAGAATCTTCAC3' |
| | 5'CGGCCAGTTGTTTTTCTGCCACCT3' |
| GAPDH | 5'GAAGGTGAAGGTCGGAGTCA3' |
| | 5'TTCACACCCATGACGAACAT3' |

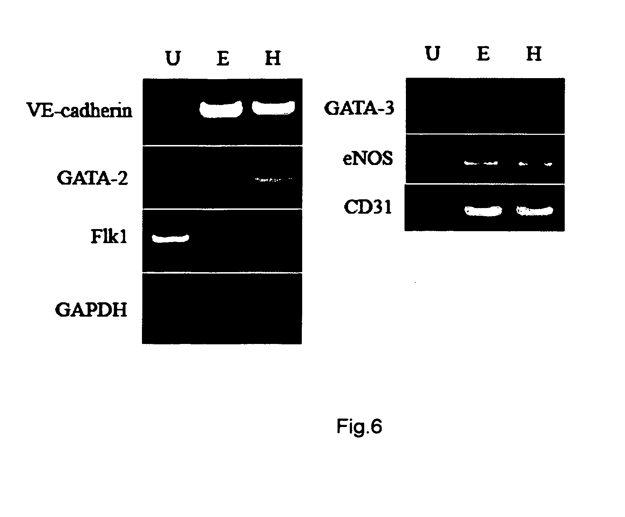

The transcription factors play an important role during embryogenesis in directing a differentiation and maintaining a phenotype, especially endothelium-specific transcription factors such as GATA-2 and -3, which are almost completely deactivated in non-differentiated hESCs, although their expression in a pure endothelial cell population derived from hESCs correlated well with their expression in HUVECs. An eNOS gene was not expressed by non-differentiated hESCs, but having been differentiated into the endothelium its expression was detected in a pure endothelial cell population at the same level as in HUVECs. Thus, the expression assay of genes characteristic for the endothelium by a semi-quantitative RT-PCR method showed the expression level for these genes in the endothelial cells derived from ESCs to be comparable to their expression in HUVECs (Fig.6).

### Genome Bisulfite Sequencing.

Genomic DNAs were isolated from non-differentiated hESCs, CD31-selected hESC-derived endothelial cells and HUVECs. A genome DNA was subjected to treatment with sodium bisulphite employing a CpGenome™ Fast DNA Modification Kit, *Chemicon.* Aliquots of the DNA modified with sodium bisulphite (25-50ng) were used for PCR in 45 amplification cycles. Primers for the PCR in the regions of interest were chosen for a sodium bisulphite-modified matrix (Table 4). Final PCR products were cloned with a pGEM-T Easy vector system (A1360, *Promega*), with plasmid clones (ten for each region) being sequenced by applying an automated ABI Prism 377 DNA Sequencer (*Applied Biosystems*).

**Table 4.**

| Gene | Sequencing of Primers | Primer position relative to a transcription start |
|---|---|---|
| eNOS | 5'TGATTTTTTGGTGGTTTTATTTTGTT3' | -321 |
| | 5'CTCTTCAAATTACCCATATTACTAT3' | 37 |
| GATA-2 | 5'AGTGTGGATGTATAGGGTGTG3' | -217 |
| | 5'ACAAACAATAAACAAACTTAAACAA3' | 67 |
| GATA-3 | 5'GATGATATTAGAAATTTTTTTAAGTTG3' | -139 |
| | 5'AATCCTCCCAAATAATTAAAAACAA3' | 252 |

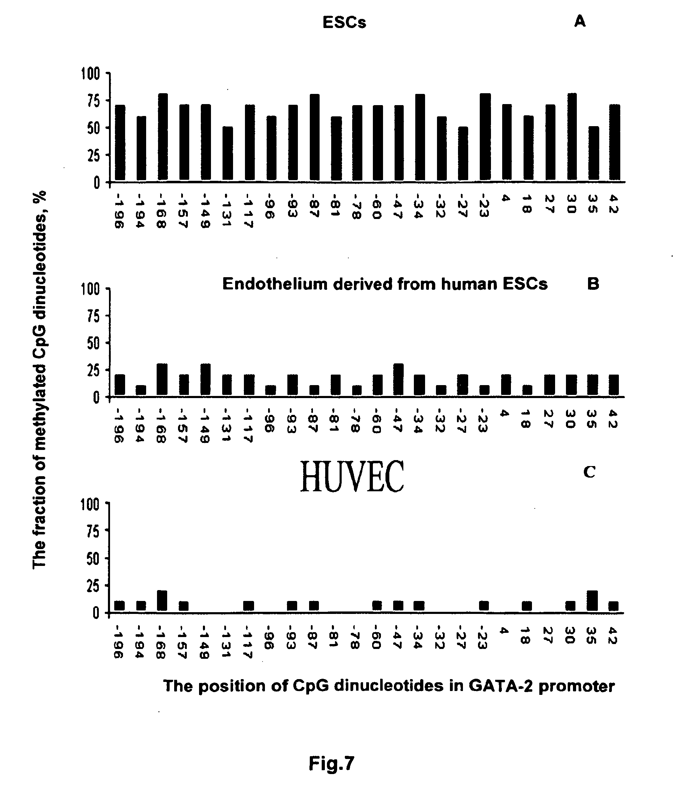

To confirm that endothelial cells differentiated from hESCs have a proper manifestation of the epigenetic state, we compared the profiles of methylation of the regulating regions of the GATA-2, -3 and eNOS genes in hESCs, endothelial cells differentiated from hESCs and HUVECs. We studied the methylation level of every CpG within the area, corresponding to a selective promoter of the GATA-2 gene (from 43rd to 187th nucleotides relative to the transcription start, considered as +1) using bisulfite sequencing. In non-differentiated hESCs this area was methylated as high as nearly 80% (Fig.7). The methylation levels of the selective promoter in HUVECs and endothelial cells differentiated from hESCs were low and ranged from 20% in endothelial cells differentiated from hESCs to 10% in HUVECs.

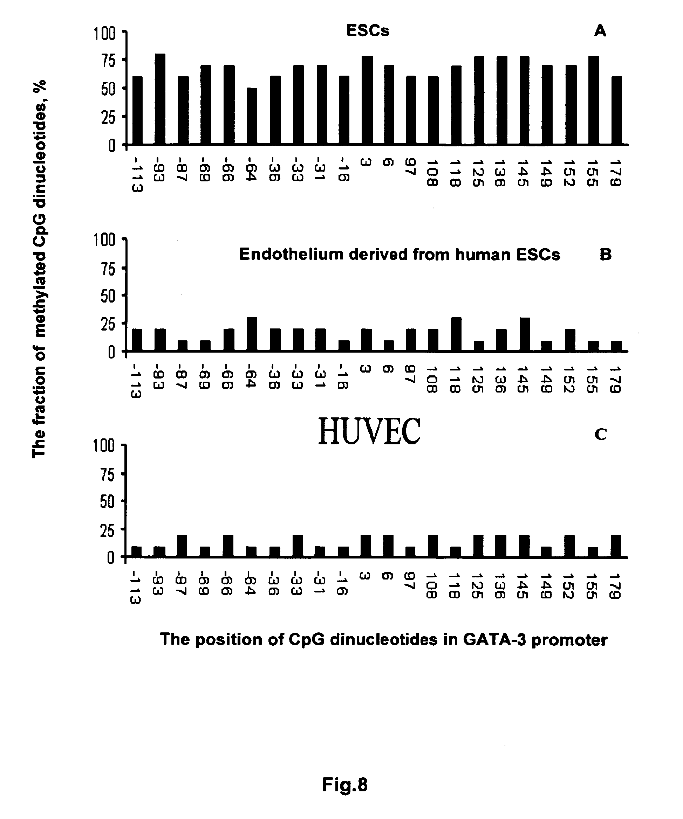

CpG located within 228-115 nucleotides were highly methylated in non-differentiated cells (up to 90%), but in hSEC-derived endothelial cells and in HUVECs the methylation of this area decreased up to 15-20% (Fig.8). Thus, in spite of hypermethylation of a proximal promoter, the GATA-3 gene had a low expression in non-differentiated hESCs, though the hypermethylated promoter increased its expression significantly.

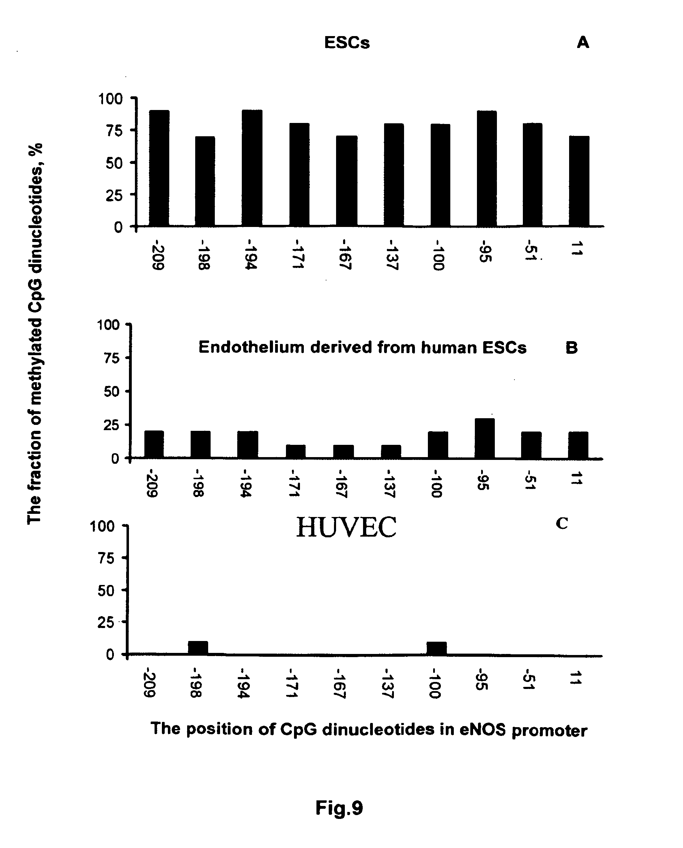

According to the data, reported by Chan (22) the most significant epigenetic modifications in the endothelial cells occur within the eNOS promoter region that is the nearest to a transcription start site. This region of the eNOS gene between 13rd and 237th nucleotides was chosen for bisulfite sequencing analysis, which showed that the region was hypermethylated in non-differentiated hESCs approximately at the same level as the regulating regions of GATA-2 and GATA-3 genes, from 70 to 90% in every CpG position. After hESCs differentiation into endothelial cells the methylation of this area reduced to 20% in functional endothelial cells selected from hESCs and did not exceed 10% in HUVECs (Fig. 9).

Bisulfite sequencing allowed detecting that the region of the GATA-2 selective promoter was hypermethylated in hESCs and hypomethylated in hESC-derived endothelial cells. Thereby we demonstrated the methylation status of the GATA-2 selective promoter influenced the expression of this transcription factor.

Taking into account the correlation of expression of the GATA-2 and its methylation promoter in hESC-derived endothelial cells we could admit an important role of the GATA-2 selective promoter in an endothelial differentiation. In spite of hypermethylation of the GATA-3 proximal promoter, the GATA-3 gene was expressed low in non-differentiated hESCs. However, we could not discard that an alternative promoter induced the GATA-3 gene in non-differentiated hESCs.

Having analyzed all the findings we can come to a conclusion that in vitro external signals give rise to a complex combination of molecular mechanisms resulting in induction of certain factors of an expression transcription. Moreover, supposing that DNA de-methylation is a final stage of epigenetic events leading to gene activation, this induction is permanent and irreversible. Stem cells are certainly to have a great potential, but this potential must be regulated in vitro otherwise there can be undesired effects under natural conditionals of differentiation.

According to our data, GATA-2 and eNOS promoters undergo hypermethylation during hESC differentiation into endothelial cells. Therefore, we can conclude that an endothelium-specific expression of eNOS is regulated by a double negative epigenetic control. In non-differentiated hESCs methylation blocked not only the eNOS promoter but also the promoter of its regulating factor of the GATA-2 transcription.

Our investigations demonstrate significant differences in methylation of promoter regions of three genes from hESCs to hESC-derived endothelial cells. However, we observed some differences between the regions of methylation promoters of the three genes studied in hESC-derived endothelial cells and HUVECs. In hESC-derived endothelial cells the promoter regions of these genes were slightly more methylated than the corresponding regions in HUVECs, though this did not result in transcription suppression. It is likely to reflect that endothelial cells originated in hESCs were not endothelial cells of the umbilical vein, rather were a mixture of specialized subtypes of endothelial cells (for example, capillary, aortic endothelial cells, endothelial cells of the lung), where the methylation level is variable.

The endothelial cells produced can be cryoconserved and stored. Herein the cells keep their viability and properties.

Cryoconservation and thawing of endothelial cells were carried out as follows.

A medium for cryconservation consisted of 90% of a fetal bovine serum (inactivated at 56° C for 45 min) (*Invitrogen* or any equivalent analogue), and 10% of DMSO (*ICN Biomedicals, Inc.* or any equivalent analogue).

The medium was removed from cells cultured, the cells were washed out with PBS twice, then 2 ml trypsin/EDTA per a 100 mm plate or a 75 cm² bottle were added. The cells were incubated at the temperature +37° C for 5 min, trypsin/EDTA was neutralized with a 5 ml cultural medium and the culture was pipetted to get a single-cell suspension. The suspension produced was put into a 25 ml glass-tube and centrifuged for 5 min at 1200 rpm. The deposition was suspended in a 1ml medium for cryoconservation, then put into cryovials and immediately kept at -70° C for 24 hours. The next day the cryovials were moved into liquid nitrogen for prolonged storage. Within one cryovial 1.5-3 million cells were cryoconserved.

Cryovials were thawed on a water-bath at +37° C and sterilized with 95% ethanol. The vial content was placed into a 15 ml glass-tube and 9ml of a cultural medium were gradually added. Then the tube was centrifuged for 5 min at 1000 rpm, the supernatant fluid was removed, the deposition was suspended in a 1ml medium and put into a culture plate or a bottle with a medium. The cells were homogeneously distributed on a surface and were incubated at +37° C and 5% CO₂.

### Methods, Techniques and Usage of Produced Endothelial Cells (endotheliocytes)

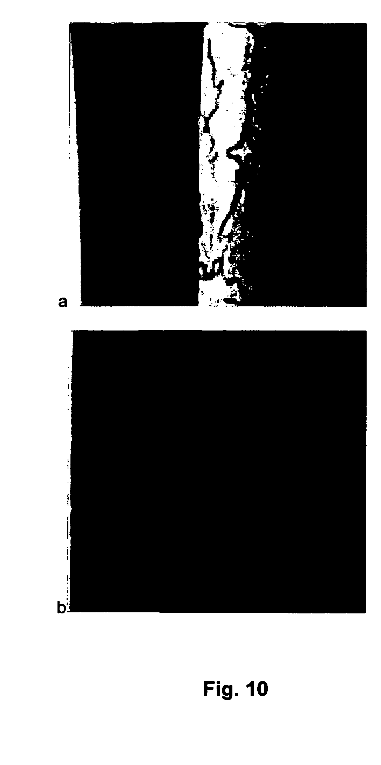

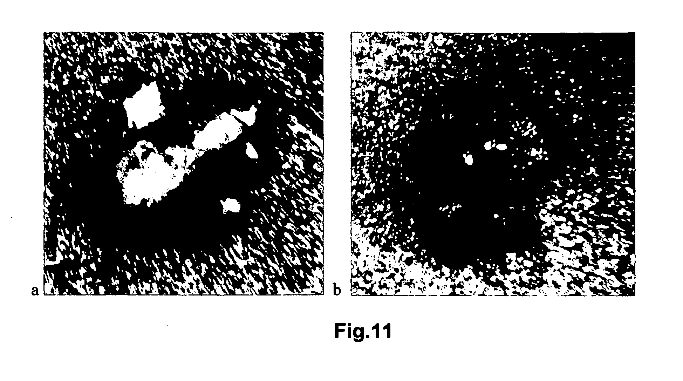

1. The endothelial cells produced with the abovementioned method can be used to form a vascular network (collateralization) de novo due to neoangiogenesis by introducing 10⁴, 10⁵, 10⁶ or more cells into a vein or an artery of mammals including man (Fig. 10).
2. The produced cells can be used for vascularization of an implant by inserting them into a biocompatible material such as gelatin, chytosan, collagen, matrigel and others or their combinations providing a formation of a 3D-structure which apart from endothelial cells may contain such cells as fibroblasts, cardiomyocytes, hepatocytes, keratinocytes, neurons, bone marrow multipotent cells, glial cells, chondrocytes, osteocytes and other cell types or cell type combinations, and for implantation to mammals including man. An implant vascularization may be achieved either by involving body's own endothelial cells or by vascularization with transplanted cells from the 3D structure to provide blood supply of an implant (an inserted tissue or cellular composition) (Fig.11).
3. The produced endothelial cells may be applied to produce a tubelike structure covered with endotheliocytes by placing them on a 2D substrate feeder made of collagen, matrigel, chytosan, fibronektines and/ or other biocompatible materials in combination with other cell types or without them. This structure can be used as a tube in vascular surgery to restore an impaired or insufficient blood circulation by replacing a part, a fragment or an entire blood vessel or stenting it.
4. The endothelial cells produced by a standard method from a standard renewable cellular culture may be used to test toxicity of chemical or biological substances or might be employed to determine an efficacy of substances to stimulate the growth (proliferation) of endothelial cells by evaluating the proliferation or death of endothelial cells by known methods.
5. Endothelial cells can be used for diagnostic or therapeutic purposes, to treat genetic disorders or to deliver toxin genes or toxins themselves into the sites of active angiogenesis (vessel growth), to block the latter (for instance, in case of vessel growth within a tumor) in order to discontinue a blood supply of an organ or tissue or for its subsequent death as a result of genetic modification by constructions containing gene-reporters, for instance, a green fluorescent protein or similar ones, or by genetic constructions containing toxin genes, or by genetic constructions containing genes to restore the function of damaged genes and introduce modified endothelial cells into mammals including man. Endothelial cells modified with genetic constructions coding growth factors may be vectors to stimulate a growth of certain tissue, cells or cells groups.
6. Combinations of the abovementioned methods of usage.

Thus, our 2D-system of differentiation allowed us to produce endothelial cells effectively, to chose a pure population of functional endothelial cells which gain the same epigenetic status of the key transcription factors and homeostasis of gene maintenance as their analogues differentiating within an embryo in vivo do. The endothelial cells produced can be widely used in clinical practice.

Selected hESC-derived endothelial cells expressed endothelial-specific genes, formed vascular-like structures and had the same structure as primary endothelial cells. CD31-selected endothelial cells differentiated from hESCs were capable to be cultured in vitro at least up to four passages.

Using our method of differentiation we observed the CD31 and VE-cadherin expression on the 6^{th} day. Undoubtedly it could result from the differences between cell lines, although culturing conditions (i.e. a culturing medium, growth factors, treatment) have a gross effect on differentiation potency. It is interesting to note that we revealed the VE-cadherin expression on the 6^{th} day of hESC differentiation, whereas under natural conditions its expression was noticed only in the 8^{th} gestation week. Thus, the VE-cadherin marker of differentiated endothelial cells was detected in vitro very early.

### References:

1. Baharvand, H., Jafary, H., Massumi, M., Ashtiani, S.K. Generation of insulin-secreting cells from human embryonic stem cells. Dev Growth Differ. 2006, 48: 323-332.
2. Hay, D.C., Zhao, D., Ross, A., Mandalam, R., Lebkowski, J., Cui, W. Direct Differentiation of Human Embryonic Stem Cells to Hepatocyte-like Cells Exhibiting Functional Activities. Cloning Stem Cells. 2007, 9: 51-62
3. Heng, B.C., Haider, H.Kh., Sim, E.K., Cao, T., Ng, S.C. Strategies for directing the differentiation of stem cells into the cardiomyogenic lineage in vitro. Cardiovasc Res. 2004, 62: 34-42.
4. Xu, C., He, J.Q., Kamp, T.J., Police, S., Hao, X., O'Sullivan, C., Carpenter, M.K., Lebkowski, J., Gold JD. Human embryonic stem cell-derived cardiomyocytes can be maintained in defined medium without serum. Stem Cells Dev. 2006, 15: 931-941.
5. Lacaud, G., Keller, G., Kouskoff, V. Tracking mesoderm formation and specification to the hemangioblast in vitro. Trends Cardiovasc Med. 2004, 14, 314-317.
6. Choi, K., Kennedy, M., Kazarov. A., Papadimitriou, J.C., Keller, G. A common precursor for hematopoietic and endothelial cells. Development. 1998, 125, 725-732.
7. Chung, Y.S., Zhang, W.J., Arentson, E., Kingsley, P.D., Palis, J., Choi, K. Lineage analysis of the hemangioblast as defined by FLK1 and SCL expression. Development. 2002, 129, 5511-5520.
8. Nishikawa, S.I., Nishikawa, S., Hirashima, M., Matsuyoshi, N., Kodama, H. Progressive lineage analysis by cell sorting and culture identifies FLKI+VE-cadherin+ cells at a diverging point of endothelial and hemopoietic lineages. Development. 1998, 125, 1747-1757.
9. Huber, T.L., Kouskoff, V., Fehling, H.J., Palis, J., Keller, G. Haemangioblast commitment is initiated in the primitive streak of the mouse embryo. Nature. 2004, 432, 625-630.
10. Cerdan, C., Rouleau, A., Bhatia, M., VEGF-A165 augments erythropoietic development from human embryonic stem cells. Blood. 2004, 103, 2504-12.
11. Chadwick, K, Wang, L., Li, L., Menendez, P., Murdoch, B., Rouleau, A., Bhatia, M. Cytokines and Bump-4 promote hematopoietic differentiation of human embryonic stem cells. Blood. 2003,102, 906-915
12. Levenberg, S., Golub, J.S., Amit, M., Itskovitz-Eldor, J., Langer, R. Endothelial cells derived from human embryonic stem cells. Proc Natl Acad Sci USA. 2002, 99: 4391-4396.
13. Ng, E.S., Davis, R.P., Azzola, L., Stanley, E.G., Elefanty, A.G. Forced aggregation of defined numbers of human embryonic stem cells into embryoid bodies fosters robust, reproducible hematopoietic differentiation. Blood. 2005, 106, 1601-1603.
14. Zambidis, E.T., Peault, B., Park, T.S., Bunz, F., Civin, C.I., Hematopoietic differentiation of human embryonic stem cells progresses through sequential hematoendothelial, primitive, and definitive stages resembling human yolk sac development. Blood. 2005, 106, 860-870.
15. Zhan, X., Dravid, G., Ye, Z., Hammond, H., Shamblott, M., Gearhart, J., Cheng, L. Functional antigen-presenting leucocytes derived from human embryonic stem cells in vitro. Lancet. 2004, 364, 163-171
16. Kaufman, D.S., Hanson, E.T., Lewis, R.L., Auerbach, R., Thomson, J.A. Hematopoietic colony-forming cells derived from human embryonic stem cells. Proc Natl Acad Sci USA. 2001, 98, 10716-10721.
17. Vodyanik, M.A., Bork, J.A., Thomson, J.A., Slukvin, I.I. Human embryonic stem cell-derived CD34+ cells: efficient production in the coculture with OP9 stromal cells and analysis of lymphohematopoietic potential. Blood. 2005,105, 617-626.
18. Hirashima, M., Kataoka, H., Nishikawa, S., Matsuyoshi, N., Nishikawa, S. Maturation of embryonic stem cells into endothelial cells in an in vitro model of vasculogenesis. Blood. 1999, 93,1253-1263.
19. Lagarkova, M.A., Volchkov, P.Y., Lyakisheva, A.V., Philonenko, E.S., Kiselev, S.L. Diverse epigenetic profile of novel human embryonic stem cell lines. Cell Cycle. 2006, 5: 416-420.
20. Gerecht-Nir, S., Ziskind, A., Cohen, S., Itskovitz-Eldor, J. Human embryonic stem cells as an in vitro model for human vascular development and the induction of vascular differentiation. Lab Invest. 2003, 83: 1811-1820.
21. Bompais, H. et al. Human endothelial cells derived from circulating progenitors display specific functional properties compared with mature vessel wall endothelial cells. Blood 103, 2577-2584 (2004)
22. Chan, Y., Fish, J.E., D'Abreo, C. The cell-specific expression of endothelial nitric-oxide synthase: a role for DNA methylation. J Biol Chem. 2004, 279: 35087-35100.

## Claims

1. A method for producing endothelial cells from human embryonic stem cells (hESCs), involving differentiation of hESCs with a human vascular endothelial growth factor (VEGF) on a substrate and in a medium that provide targeted direct differentiation of hESCs into endothelial cells and separation of the produced endothelial cells, **characterized in that** a synthetic medium for differentiation is used, which is based on a mixture of DMEM/F12 and a KO serum substitute in a ratio: 75-95% liquid DMEM/F12, 5-25% liquid KO serum substitute, wherein the synthetic medium additionally contains SCF, BMP4, bFGF, TGFbeta growth factors in effective amounts, and wherein the TGFbeta is added on the 3^{rd}-6^{th} differentiation day and the separation is carried out on the 3^{rd} **-** 9^{th} day by a method of immunological selection using endothelial cell specific markers, whereafter the produced endothelial cells are cultured in said synthetic medium at a density of plating not less than 50.000 cells per 1 cm².

2. A method for producing endothelial cells from human embryonic stem cells (hESCs), involving differentiation of hESCs with a human vascular endothelial growth factor (VEGF) on a substrate and in a medium that provide targeted direct differentiation of hESCs into endothelial cells and separation of the produced endothelial cells, **characterized in that** a medium for differentiation is used which is based on a mixture of DMEM/F12 and a fetal bovine serum in the ratio: 75-95% liquid DMEM/F12 -, 5-25% liquid fetal bovine serum, wherein the medium additionally contains SCF, bFGF growth factors in effective amounts, and wherein the separation is carried out on the 3^{rd} -9^{th} day by a method of immunological selection using endothelial cell specific markers, whereafter the produced endothelial cells are cultured in said medium at a density of plating not less than 50.000 cells per 1 cm².

## Patentansprüche

1. Verfahren zur Herstellung von Endothelzellen aus humanen embryonalen Stammzellen (hESCs), umfassend die Differenzierung von hESCs mit einem humanen vaskularen endothelialen Wachstumsfaktor (VEGF) auf einem Substrat und in einem Medium, das zu einer zielgerichteten Differenzierung von hESCs zu Endothelzellen führt und Abtrennung der hergestellten Endothelzellen, **dadurch gekennzeichnet, dass** für die Differenzierung ein synthetisches Medium verwendet wird, welches auf einer Mischung von DMEM/F12 und einem KO-Serumersatz in einem Verhältnis von 75-95% DMEM/F12-Flüssigkeit und 5-25% KO-Serumersatzflüssigkeit beruht, wobei das synthetische Medium des Weiteren SCF-, BMP4-, bFGF-, TGFbeta-Wachstumsfaktoren in wirksamen Mengen umfasst, und wobei das TGFbeta am dritten bis sechsten Differenzierungstag hinzugefügt wird und die Abtrennung am dritten bis neunten Tag durch ein immunologisches Selektionsverfahren unter Verwendung Endothelzellen-spezifischer Marker erfolgt, woraufhin die hergestellten Endothelzellen in besagtem synthetischen Medium mit einer Plattierungsdichte von nicht weniger als 50000 Zellen pro 1 cm² kultiviert werden.

2. Verfahren zur Herstellung von Endothelzellen aus humanen embryonalen Stammzellen (hESCs), umfassend die Differenzierung von hESCs mit einem humanen vaskularen endothelialen Wachstumsfaktor (VEGF) auf einem Substrat und in einem Medium, das zu einer zielgerichteten Differenzierung von hESCs zu Endothelzellen führt und Abtrennung der hergestellten Endothelzellen, **dadurch gekennzeichnet, dass** für die Differenzierung ein Medium verwendet wird, welches auf einer Mischung von DMEM/F12 und einem fetalem Kälberserum in einem Verhältnis von 75-95% DMEM/F12-Flüssigkeit und 5-25% fetaler Kälberserumflüssigkeit beruht, wobei das Medium des Weiteren SCF-, bFGF-Wachstumsfaktoren in wirksamen Mengen umfasst, und wobei die Abtrennung am dritten bis neunten Tag durch ein immunologisches Selektionsverfahren unter Verwendung Endothelzellen-spezifischer Marker erfolgt, woraufhin die hergestellten Endothelzellen in besagtem Medium mit einer Plattierungsdichte von nicht weniger als 50000 Zellen pro 1 cm² kultiviert werden.

## Revendications

1. Procédé de production de cellules endothéliales à partir de cellules souches embryonnaires humaines (CSEh), impliquant la différenciation des CSEh avec un facteur de croissance de l'endothélium vasculaire (VEGF) humain sur un substrat et dans un milieu qui permet d'obtenir une différenciation directe ciblée des CSEh en cellules endothéliales et une séparation des cellules endothéliales produites, **caractérisé en ce qu'**un milieu de différenciation synthétique est utilisé, qui est basé sur un mélange de DMEM/F12 et de substitut de sérum KO selon un rapport : DMEM/F12 liquide à 75-95 %, substitut de sérum KO liquide à 5-25 %, où le milieu synthétique contient en outre les facteurs de croissance SCF, BMP4, bFGF, et TGFbêta en des quantités efficaces, et où le TGFbêta est ajouté au 3e-6e jour de différenciation et la séparation est réalisée au 3e-9e jour par un procédé de sélection immunologique en utilisant des marqueurs spécifiques aux cellules endothéliales, après quoi les cellules endothéliales produites sont mises en culture dans ledit milieu synthétique à une densité d'ensemencement minimale de 50 000 cellules pour 1 cm2.

2. Procédé de production de cellules endothéliales à partir de cellules souches embryonnaires humaines (CSEh), impliquant la différenciation des CSEh avec un facteur de croissance de l'endothélium vasculaire (VEGF) humain sur un substrat et dans un milieu qui permet d'obtenir une différenciation directe ciblée des CSEh en cellules endothéliales et une séparation des cellules endothéliales produites, **caractérisé en ce qu'**un milieu de différenciation est utilisé, qui est basé sur un mélange de DMEM/F12 et de sérum bovin foetal selon le rapport : DMEM/F12 liquide à 75-95 %, sérum bovin foetal liquide à 5-25 %, où le milieu contient en outre les facteurs de croissance SCF, bFGF en des quantités efficaces, et où la séparation est réalisée au 3e-9e jour par un procédé de sélection immunologique en utilisant des marqueurs spécifiques aux cellules endothéliales, après quoi les cellules endothéliales produites sont mises en culture dans ledit milieu à une densité d'ensemencement minimale de 50 000 cellules pour 1 cm2.
